# EUROPEAN PATENT APPLICATION

(11) **EP 4 342 423 A1**
(43) Date of publication of application: **27.03.2024**
(21) Application number: 21940537.0
(22) Date of filing: 26.11.2021
(51) Int. Cl.: A61F 2/24

(54) **TRICUSPID VALVE FRAME AND VALVE PROSTHESIS THEREOF**

(30) Priority: 17.05.2021 CN 202110535980
(71) Applicant: Kingstronbio (Changshu) Co., Ltd., Changshu Jiangsu 215500 (CN)
(72) Inventor: ZHONG, Shengping, Changshu, Jiangsu 215500 (CN); JIN, Yongfu, Changshu, Jiangsu 215500 (CN)
(74) Representative: Luppi Intellectual Property S.r.l.
(86) International application number: PCT/CN2021/133518
(87) International publication number: WO 2022/242093

(57) **Abstract**

The present disclosure relates to a tricuspid valve frame and a valve prosthesis thereof. The tricuspid valve frame includes an inner frame part and an outer frame part. An end of the inner frame part is formed into an inflow end. An end of the outer frame part is formed into an outflow end. Another end of the inner frame part away from the inflow end is fixedly connected to another end of the outer frame part away from the outflow end. In an expanding state, the inflow end has a smaller radial size than the outflow end. The inflow end in the expanding state forms a support edge everting towards a side away from an axis of the tricuspid valve frame. In the present disclosure, the support edge is designed, so that the valve frame can be reliably supported on a valve annulus. Meanwhile, by using a structural form that the outer frame part and the inner frame part are processed separately, the molding of connecting claws are facilitated. In addition, the radial size of the inflow end is smaller than the radial size of the outflow end, it can be ensured that a force transmitted to the valve annulus does not have adverse effects on the valve annulus, and the valve prosthesis can also be prevented from falling off at the same time.

## Description

### TECHNICAL FIELD

The present disclosure relates to the field of medical instrument technologies, and in particular, to a tricuspid valve frame and a valve prosthesis thereof.

### BACKGROUND

An artificial valve is implanted into a body through intervention means, that is, the artificial valve is delivered to an implantation site via a delivery system, the valve is then unfolded through its self-expanding properties or by balloon dilation, and is riveted firmly onto the corresponding valve annulus.

An aortic intervention valve developed rapidly is achieved by riveting firmly onto the aortic valve annulus by means of radial support force.

The human heart has four valves in total: an aortic valve, a mitral valve, a pulmonary valve, and a tricuspid valve. The tricuspid valve is located on the valve annulus between an atrium and a ventricle of a right heart. Due to the softness of the tricuspid valve annulus, the valve cannot be riveted firmly by a radial support force. Meanwhile, due to the complex structure around the tricuspid valve annulus and the presence of numerous nervous systems, if the force of the valve is delivered to these tissues, resulting in malignant accidents where the heart cannot work. However, due to the periodic blood pressure on the valve during operation, a stable riveting method is necessary. Therefore, it is necessary to develop a tricuspid valve frame and a valve prosthesis that can solve the above problems.

### SUMMARY

The present disclosure relates to a tricuspid valve frame and valve prosthesis thereof to solve the problem that the tricuspid valve annulus is unable to rivet the valve with radial support force in the related art.

In a first aspect of the present disclosure, a tricuspid valve frame is provided. The tricuspid valve frame includes: an inner frame part and an outer frame part. An end of the inner frame part is formed into an inflow end; an end of the outer frame part is formed into an outflow end; and another end of the inner frame part away from the inflow end is fixedly connected to another end of the outer frame part away from the outflow end.

In an expanding state, the inflow end has a smaller radial size than the outflow end.

The inflow end in the expanding state forms a support edge everting towards a side away from an axis of the tricuspid valve frame.

In an embodiment, the outer frame part includes a first frame body and a fixing claw. The fixing claw is fixedly provided on the first frame body, and a space for fixing a valve annulus is maintained between the fixing claw and the support edge.

In an embodiment, at least two fixing claws are provided in a circumferential direction of the outer frame part, and the at least two fixing claws are wrapped and connected to each other by fabric.

In an embodiment, the inner frame part is formed by interweaving multiple first connecting beams, the outer frame part is formed by interweaving multiple second connecting beams, and a ratio of a sectional area of the first connecting beams to a sectional area of the second connecting beams is 1.2 to 3.0.

In an embodiment, the inner frame part and the outer frame part are connected by binding metal or non-metal wire, or the inner frame part and the outer frame part are connected by welding.

In an embodiment, the inner frame part includes a second frame body and a connecting claw. An end of the connecting claw is fixed to the second frame body, and another end of the connecting claw passes through the outer frame part and extends from the outflow end.

In an embodiment, at least two connecting claws are provided, and the at least two connecting claws are retracted toward an axis of the valve frame.

In an embodiment, the second frame body and the connecting claws form an integrated structure.

In an embodiment, the outer frame part is fixed on an outer side of the inner frame part.

In another aspect of the present disclosure, a tricuspid valve prosthesis is provided. The tricuspid valve prosthesis includes a tricuspid valve frame provided in the first aspect of the present disclosure. The tricuspid valve prosthesis further includes a valve skirt, a leaflet, and a sealing skirt. The valve skirt is enclosed on an inner side of the inner frame part and the outer frame part, and the leaflet is fixed on an inner side of the valve skirt, and the sealing skirt is enclosed on an outer side of a bottom end of the outer frame part.

The technical solution provided in the present disclosure can achieve the following beneficial effects:

The present disclosure provides a tricuspid valve frame and valve prosthesis thereof. The support edge is designed, so that the valve frame can be reliably supported on a valve annulus. Meanwhile, by using a structural form that the outer frame part and the inner frame part are processed separately, the molding of connecting claws are facilitated. In addition, the radial size of the inflow end is smaller than the radial size of the outflow end, it can be ensured that a force transmitted to the valve annulus does not have adverse effects on the valve annulus, and the valve prosthesis can also be prevented from falling off at the same time.

It should be understood that the above general description and the following detailed description are only exemplary, which cannot limit the present disclosure.

### BRIEF DESCRIPTION OF DRAWINGS

FIG. 1 is a structural schematic diagram of a tricuspid valve frame according an embodiment of the present disclosure;
FIG. 2 is a structural schematic diagram of an inner frame part;
FIG. 3 is a structural schematic diagram of an outer frame part;
FIG. 4 is a structural schematic diagram of a tricuspid valve frame according an embodiment of the present disclosure; and
FIG. 5 is a schematic diagram of the tricuspid valve frame after implantation into the human body according an embodiment of the present disclosure.

### Reference signs:

1 - tricuspid valve frame;
   11 - inner frame part;
      111 - inflow end;
      112 - second frame body;
   12 - outer frame part;
      121 - outflow end;
      122 - first frame body;
      123 - support edge;
      124 - space;
   13- fixing claw;
   14 - connecting claw;
2 - leaflet;
3 - valve skirt;
4 - primary leaflet;
5 - valve annulus;
6 - ventricle; and
7- atrium.

The accompanying drawings herein are incorporated into, and constitute a part of, this specification, illustrate embodiments consistent with the present disclosure, and serve to explain the principles of the present disclosure together with this specification.

### DESCRIPTION OF EMBODIMENTS

In order to better illustrate objectives, technical solutions and advantages of the present disclosure, the present disclosure will be further described in detail below with reference to the drawings and embodiments. It should be understood that the specific embodiments described herein are used just to explain the present disclosure, but not to limit the present disclosure.

In the description of the present disclosure, unless otherwise expressly stipulated and defined, the terms such as "first" and "second" are merely used for descriptive purposes, but shall not be illustrated as indicating or implying relative importance. Unless otherwise specified or stated, the term "multiple" refers to two or more; the terms such as "connection", "fixed/fixation", etc. shall be illustrated in a broad sense. For example, "connection" may refer to "fixedly connection", "detachably connection", "integrally connection", or "electrically connection"; and/or directly connection or indirectly connection through an intermediary. For one skilled in the art, the specific meanings of the above terms in the present disclosure can be understood according to specific circumstances.

In the description of this specification, it should be understood that the directional words such as "above/upper" and "below/lower" described in embodiments of the present disclosure are described from the perspective shown in the drawings, but cannot be illustrated as a limitation to the embodiments of the present disclosure. In addition, it should be understood in the context that an element is connected "above" or "below" another element, the element may be directly connected "above" or "below" another element, or the element may be indirectly connected "above" or "below" the another element through an intermediate element.

A tricuspid valve is a crucial component of human heart, and is located on the annulus between an atrium and a ventricle of the right heart, ensuring that blood circulation flows from a right atrium to a right ventricle and passes through a certain flow.

When a primary tricuspid valve experiences dysfunction, an artificial valve can be implanted into the body through an intervention means to replace the function of the primary tricuspid valve. The intervention means involves transporting the artificial valve to the implantation site through a delivery system. The valve is then unfolded either through its self-expanding properties or by balloon dilation, and is riveted onto the corresponding valve annulus.

However, due to the softness of the tricuspid valve annulus, the artificial valve cannot be riveted with radial support force, which can easily lead to the risk of valve prosthesis detachment and touching the nervous system by the valve prosthesis.

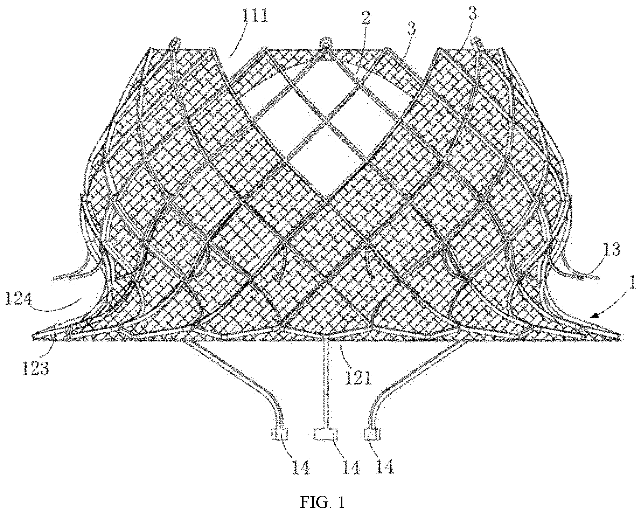

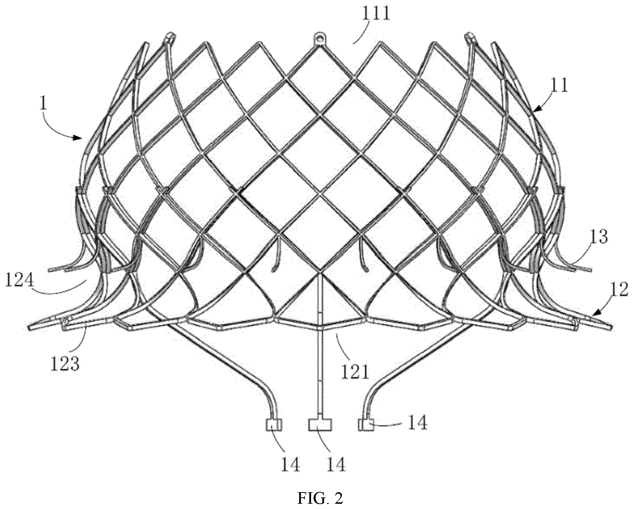

For this purpose, as shown in FIG. 1 to FIG. 5, this embodiment provides a tricuspid valve frame 1 and a tricuspid valve prosthesis including the tricuspid valve frame 1. The tricuspid valve prosthesis further includes a valve skirt 3 and a leaflet 2. The valve skirt 3 is enclosed on an inner side of an inner frame part 11 and an outer frame part 12, and the leaflet 2 is fixed on an inner side of the valve skirt 3.

The tricuspid valve frame 1 includes an inner frame part 11 and an outer frame part 12. An end of the inner frame part 11 is formed into an inflow end 111, and an end of the outer frame part 12 is formed into an outflow end 121. Another end of the inner frame part 11 away from the inflow end 111 is fixedly connected to another end of the outer frame part 12 away from the outflow end 121. In an expanding state, a radial size of the inflow end 111 is smaller than a radial size of the outflow end 121. The inflow end 121 in the expanding state forms a support edge 123 everting towards a side away from an axis of the tricuspid valve frame.

The inner frame part 11 and the outer frame part 12 may be made of metal tubes. For example, the metal tubes may be cut by using a cutting process, and then the cut metal tubes may be stretched through a stretching process to form the inner frame part 11 and outer frame part 12 that are stretched. When the tricuspid valve frame is implanted into a human body through a delivery system, the tricuspid valve frame is retracted within the delivery system. At an implantation position in the human body, the tricuspid valve frame is released and automatically expands, and is supported by the support edge 123 to a side of a valve annulus 5 facing the ventricle 6, thereby completing the implantation of the tricuspid valve frame.

The inflow end 111 with a smaller diameter can extend into the atrium 7, and the outflow end 121 with a larger diameter is located in the ventricle 6. The outflow end 121 can stretch the primary leaflet 4 after the valve prosthesis is implanted in the human body, and can be riveted onto the primary leaflet 4. When the ventricle 6 contracts, the valve prosthesis is closed, and the valve prosthesis is pushed by blood to act on the primary leaflet 4, and then acts on the valve annulus 5 through the primary leaflet 4. At this time, the force transmitted to the valve annulus 5 will not have a negative impact on the valve annulus 5, and the valve prosthesis can work smoothly. Meanwhile, in the expanding state, since the outflow end 121 of the outer frame part 12 has a larger diameter, and the support edge 123 can abut against a side of the valve annulus 5 facing the ventricle 6, the accident of falling off the valve prosthesis caused by the systolic pressure of the ventricle 6 pushing the valve prosthesis into the atrium 7 will be avoided.

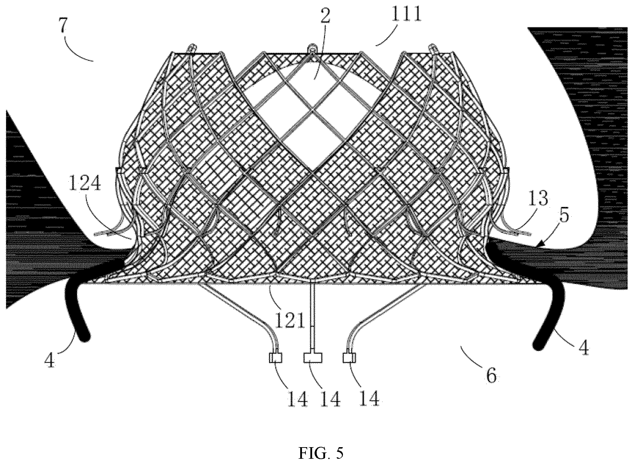

It should be noted that due to the softness of the primary leaflet 4 in the human body, the outflow end 121 of the valve prosthesis in the present disclosure is further designed to be relatively soft, so that the valve prosthesis can be fully fitted to the primary leaflet 4, as shown in FIG. 5. Moreover, the primary leaflet 4 is stretched around the outer side of the valve prosthesis to prevent paravalvular leakage, and meanwhile, the stress on the primary leaflet 4 can be more uniform, which better conforms to the stress state of the primary leaflet 4. Meanwhile, the valve prosthesis can further be prevented from damaging the primary leaflet 4.

In addition, the outer frame part 12 and the inner frame part 11 may be processed separately, and then the inner frame part 11 and the outer frame part 12 may be assembled to form a complete valve frame.

In order to ensure reliable connection between the inner frame part 11 and the outer frame part 12, the inner frame part 11 and the outer frame part 12 may be connected by binding metal or non-metal wire, or the inner frame part 11 and the outer frame part 12 may connected by welding.

It should be noted that the diameter of the inner frame part 11 is relatively small, and the overall structure of the inner frame part 11 may have relatively high stability and deformation resistance, thereby improving the reliability of fixing the leaflet 2 by the inner frame part 11. Since the diameter of the outer frame part 12 is relatively large, the structural flexibility of the outer frame part 12 can be improved, and when the outer frame part 12 is in contact with the valve annulus 5, damage to the valve annulus 5 can be avoided.

In this embodiment, the inner frame part 11 is formed by interweaving multiple first connecting beams, and the outer frame part 12 is formed by interweaving multiple second connecting beams, so that the inner frame part 11 and the outer frame part 12 forms a network structure. A ratio of a sectional area of the first connecting beams to a sectional area of the second connecting beams is 1.2 to 3.0. The total number of the first connecting beams may be the same as the total number of the second connecting beams, and the first connecting beams are relatively thin, so that the inner frame part 11 can be more easily and fully fitted with the primary leaflet 4 without causing damage to the primary leaflet 4.

As a specific implementation, the outer frame part 12 includes a first frame body 122 and a fixing claw 13. The outflow end 121 is formed as an end of the first frame body 122, and the fixing claw 13 is fixedly provided on the first frame body 122. The fixing claw 13 protrudes from an outer surface of the first frame body 122, so that a space 124 is maintained between the fixing claw 13 and the support edge 123 for fixing a valve annulus 5. It should be noted that a distance size of the space 124 in an axial direction of the valve frame is greater than a thickness of the valve annulus 5. After the support edge 123 abuts against a surface of the valve annulus 5, a gap is maintained between another surface of the valve annulus 5 and the fixing claw, so that when the valve prosthesis is closed by a blood flow force, the valve annulus 5 dose not contact the fixing claw frequently and is not damaged. However, when the valve prosthesis has a tendency to move towards the ventricle 6 under the action of the blood flow, the fixing claw can abut against a surface of the valve annulus 5 facing away from the ventricle 6, thereby preventing the valve prosthesis from falling into the ventricle 6.

Multiple fixing claws 13 are uniformly arranged in a circumferential direction of the outer frame part 12, so that the valve annulus 5 can be uniformly stressed when the fixing claws 13 abut against the valve annulus 5, thereby reducing the pressure on the valve annulus 5. In addition, multiple fixing claws 13 may be wrapped and connected by fabric, on one hand, the stability of the fixing claws 13 is improved, on the other hand, the fixing claws 13 are prevented from puncturing the valve annulus 5, while facilitating tissue growth on the valve annulus 5.

As a specific implementation, the inner frame part 11 includes a second frame body 112 and a connecting claw 14. An end of the connecting claw 14 is fixed to the second frame body 112, and another end of the connecting claw 14 passes through the outer frame part 12 and extends from the outflow end 121 of the outer frame part 12.

The valve prosthesis in this embodiment is transported to the human body through a dedicated delivery system. The tricuspid valve frame 1 is compressed into the delivery system as a whole through radial contraction of the tricuspid valve frame 1, and is connected to the delivery system through the connecting claws 14. After entering a set position inside the human body, the delivery system releases the valve prosthesis. At this time, the connecting claw 14 is separated from the delivery system, and the tricuspid valve frame 1 is riveted to the valve annulus by radial expansion itself.

Multiple connecting claws 14 are provided, and multiple connecting claws 14 are retracted toward an axis of the valve frame. And there is a space between the connecting claws 14, so that an operating space can be reserved for the next interventional implantation of the valve prosthesis.

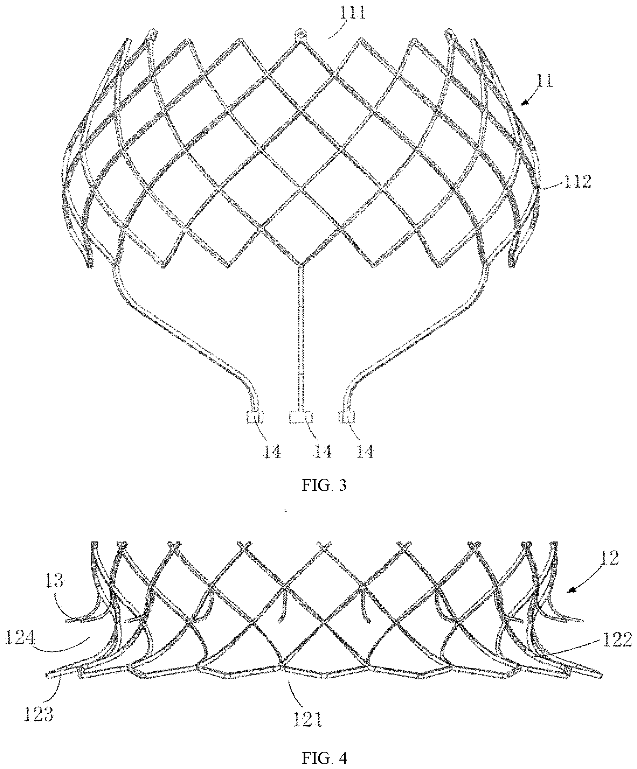

In addition, it should be noted that for processing the valve frame by laser processing, if the inner frame part 11 and the outer frame part 12 are integrally processed, the connecting claw 14 will not be obtained by directly processing. The connecting claw 14 needs to be welded separately, which is a complex process and difficult to ensure the processing accuracy of the connecting claw 14. Therefore, in this embodiment, as shown in FIG. 3, the inner frame part 11 and the outer frame part 12 are processed as independent parts, respectively, so that the connecting claw 14 can be directly formed on the inner frame part 11 without separately processing the connecting claw 14, thereby making the process simple and improving the processing accuracy.

It should be noted that the outer frame part 12 is fixed to an outer side of the inner frame part 11, so that when the inner frame part 11 is connected to the outer frame part 12, the placement of the connecting claws 14 is facilitated.

As a specific implementation, the tricuspid valve prosthesis provided in the present embodiment further includes a sealing skirt, and the sealing skirt is enclosed on an outer side of a bottom end of the outer frame part 12. In this embodiment, the bottom end is the outflow end 121 of the outer frame part 12. The sealing skirt may be sewn on the outer frame part 12 to prevent peripheral leakage.

In the tricuspid valve frame and valve prosthesis thereof provided by the embodiments of the present disclosure, the support edge is designed, so that the valve frame can be reliably supported on a valve annulus. Meanwhile, by using a structural form that the outer frame part and the inner frame part are processed separately, the molding of connecting claws are facilitated. In addition, the radial size of the inflow end is smaller than the radial size of the outflow end, it can be ensured that a force transmitted to the valve annulus does not have adverse effects on the valve annulus, and the valve prosthesis can also be prevented from falling off at the same time.

The above embodiments of the present disclosure are several preferred embodiments, but not intended to limit the scope of the claims. Various changes and modifications can be made by those skilled in the art without departing from the scope of the present application. Any modification, equivalent replacement, improvement, and the like made within the spirit and principle of the present disclosure shall be included in the protection scope of the present disclosure.

## Claims

1. A tricuspid valve frame, comprising:
an inner frame part (11) and an outer frame part (12);
wherein an end of the inner frame part (11) is formed into an inflow end (111); an end of the outer frame part (12) is formed into an outflow end (121); another end of the inner frame part (11) away from the inflow end (111) is fixedly connected to another end of the outer frame part (12) away from the outflow end (121);
in an expanding state, the inflow end (111) has a smaller radial size than the outflow end (121); and
the inflow end (121) in the expanding state forms a support edge (123) everting towards a side away from an axis of the tricuspid valve frame.

2. The tricuspid valve frame according to claim 1, wherein the outer frame part (12) comprises a first frame body (122) and a fixing claw (13), and the fixing claw (13) is fixedly provided on the first frame body (122), and a space (124) for fixing a valve annulus is maintained between the fixing claw (13) and the support edge (123).

3. The tricuspid valve frame according to claim 2, wherein at least two fixing claws (13) are provided in a circumferential direction of the outer frame part (12), and the at least two fixing claws (13) are wrapped and connected to each other by fabric.

4. The tricuspid valve frame according to claim 1, wherein the inner frame part (11) is formed by interweaving first connecting beams, the outer frame part (12) is formed by interweaving second connecting beams, and a ratio of a sectional area of the first connecting beams to a sectional area of the second connecting beams is 1.2 to 3.0.

5. The tricuspid valve frame according to claim 1, wherein the inner frame part (11) and the outer frame part (12) are connected by binding metal or non-metal wires, or the inner frame part (11) and the outer frame part (12) are connected by welding.

6. The tricuspid valve frame according to claim 1, wherein the inner frame part (11) comprises a second frame body (112) and a connecting claw (14), and an end of the connecting claw (14) is fixed to the second frame body (112), and the other end of the connecting claw (14) passes through the outer frame part (12) and extends from the outflow end (121).

7. The tricuspid valve frame according to claim 6, wherein at least two connecting claws (14) are provided, and the at least two connecting claws (14) are retracted toward an axis of the valve frame.

8. The tricuspid valve frame according to claim 6, wherein the second frame body (112) and the connecting claw (14) form an integrated structure.

9. The tricuspid valve frame according to claim 6, wherein the outer frame part (12) is fixed on an outer side of the inner frame part (11).

10. A tricuspid valve prosthesis, comprising:
a tricuspid valve frame (1) according to any one of claims 1-9;
a valve skirt (3);
a leaflet (2); and
and a sealing skirt,
wherein the valve skirt (3) is enclosed on an inner side of an inner frame part (11) and an outer frame part (12), and the leaflet (2) is fixed on an inner side of the valve skirt (3), and the sealing skirt is enclosed on an outer side of a bottom end of the outer frame part (12).
